# EUROPÄISCHE PATENTSCHRIFT

(11) **EP 1 898 788 B1**
(45) Veröffentlichungstag und Bekanntmachung des Hinweises auf die Patenterteilung: **13.05.2015**
(21) Anmeldenummer: 06761733.2
(22) Anmeldetag: 29.06.2006
(51) Int. Cl.: A61B 5/103, A61F 2/76

(54) **ORTHOPÄDIETECHNISCHES HILFSMITTEL, INSBESONDERE PROTHESE FÜR EINE EXTREMITÄT**
TECHNICAL ORTHOPAEDIC AUXILIARY AGENT, IN PARTICULAR PROSTHESIS FOR AN EXTREMITY
MOYEN AUXILIAIRE DE TECHNIQUE ORTHOPEDIQUE, EN PARTICULIER PROTHESE POUR UNE EXTREMITE

(30) Priorität: 01.07.2005 DE 102005031185
(43) Veröffentlichungstag der Anmeldung: 19.03.2008
(73) Patentinhaber: Otto Bock HealthCare GmbH, 37115 Duderstadt (DE)
(72) Erfinder: PUSCH, Martin, 37115 Duderstadt (DE)
(74) Vertreter: Lins, Edgar
(86) Internationale Anmeldenummer: PCT/DE2006/001129
(87) Internationale Veröffentlichungsnummer: WO 2007/003169

(56) Entgegenhaltungen:
- EP-A- 0 449 799
- EP-A2- 0 663 181
- WO-A-96/00540
- DE-B- 1 291 855
- DE-U1- 9 104 823
- DE-U1- 29 601 359
- FR-A- 1 421 562
- BLUMENTRITT S ET AL: "Die Bedeutung des statischen Prothesenaufbaus für das Stehen und Gehen des Unterschenkelamputierten" DER ORTHOPÄDE MAR 2001, Bd. 30, Nr. 3, März 2001 (2001-03), Seiten 161-168, XP002425277 ISSN: 0085-4530
- SCHERER H.W.: "Praktische Erfahrungen mit dem Aufbaumessgerät "L.A.S.A.R.-Posture"" ORTHOPÄDIE-TECHNIK, Bd. 6/99, Juni 1999 (1999-06), Seiten 468-474, XP002425278 http://www.ot-forum.de/OT/split1999/ot1999 .278-281.pdf

## Beschreibung

Die Erfindung betrifft ein orthopädietechnisches Hilfsmittel zur Überprüfung der mit einem justierbaren Element herstellbaren Sollausrichtung an einem individuellen Patienten mit Hilfe einer Detektoranordnung.

Die Anpassung orthopädietechnischer Hilfsmittel, wie Orthesen und Prothesen, erfordert eine besondere Sorgfalt, insbesondere wenn untere Extremitäten betroffen sind. Nicht korrekt eingestellte Prothesen können erhebliche Unsicherheiten des Protheseträgers bewirken und im schlimmsten Fall sogar Stürze und daraus resultierende Verletzungen verursachen.

Für die Anpassung von Orthesen und insbesondere Prothesen sind Vermessungseinrichtungen bekannt, die den Sitz des orthopädietechnischen Hilfsmittels während einer stabilen statischen Belastungsposition ermitteln, indem die auf eine Standfläche einwirkenden Kräfte elektrisch gemessen, ausgewertet und zu einer geeigneten Anzeige gebracht werden. Ein derartiges System der Anmelderin ist als LASAR Posture bekannt und im Gebrauch. Eine ähnliche Vermessungseinrichtung ist ferner durch WO 96/00540 A1 bekannt, auf der der Patient mit einer angepassten Prothese so positioniert wird, dass er auf zwei Plattformen nach Art einer Waage steht und Belastungssensoren der Plattformen eine ungleichmäßige Belastung feststellen, wenn die Prothese noch nicht richtig justiert ist. Ergänzt wird das Sensorsystem durch einen orthopädischen Gürtel mit einem Sensor, der die Beckenstellung überprüft. Problematisch ist jedoch, dass nicht jedem Orthopädiemechaniker eine derartige Einrichtung zur Verfügung steht.

Ein anderes System das zur Überprüfung der Ausrichtung eines Prothesenschaftes dient, ist durch EP 0 449 799 A1 bekannt. Die Vorrichtung enthält eine einstellbare Trageinrichtung für den Prothesenschaft, der somit beispielsweise einen Oberschenkelstumpf eines auf einer Standebene stehenden Patienten aufnehmen kann. Durch kontrollierte Bewegungen um drei senkrecht aufeinander stehenden Drehachsen werden Grenzwinkel bestimmt, innerhalb derer der Patient bei der Bewegung des Schaftes schmerzfrei bleibt. In den Drehachsen können Drehmomentgeber vorgesehen sein, durch die die Grenzen des als schmerzfrei empfundenen Bewegungsbereichs nicht nur durch Winkel um die Drehachsen sondern auch durch in den Drehachsen auftretende Drehmomente charakterisierbar sind.

FR 1 421 562 offenbart eine Prothese, die beispielsweise zwischen einem Prothesenfuß und einem mit einem Schaft zur Stumpfaufnahme verbundenen Prothesenunterschenkel eine Justieranordnung aufweist. Die Justieranordnung besteht aus zwei über ein mittiges Kugelgelenk miteinander verbundene Platten, zwischen denen vier gleichmäßig über den Umfang verteilte Schraubenanordnungen positioniert sind, sodass die obere Platte relativ zur unteren Platte in zwei Achsen der durch die untere Platte aufgespannten Ebene kippbar ist. Die Einstellung erfolgt dadurch, dass die Person mit einem über den künstlichen Fuß gezogenen Schuh Schritte geht und die Bewegung dabei beobachtet wird. Hierfür können die Schrauben entsprechend verstellt werden, um unterschiedliche Kipppositionen zu ermöglichen. Wenn dann die als richtig erkannte Sollausrichtung erreicht ist, wird die Einstellanordnung aus der Prothese entfernt und durch ein der ermittelten Position der Platte entsprechendes massives Holzteil in die Prothese eingesetzt, das somit die Prothese gemäß der ermittelten Position fest ausrichtet. Die Überprüfung der richtigen Ausrichtung der Prothese erfolgt dabei ohne Vermessungseinrichtung ausschließlich durch Beurteilung durch den Orthopädiemechaniker und/oder den Patienten. Ob dabei eine korrekte Sollausrichtung der Prothese erreicht wird, hängt entscheidend von den Erfahrungen und Beurteilungen durch den Orthopädiemechaniker ab und erweist sich häufig als fehlerhaft. Diesem Missstand ist durch die Einführung der oben erwähnten Vermessungseinrichtung LASAR Posture begegnet worden.

Der vorliegenden Erfindung liegt daher die Aufgabe zugrunde, bestimmte Justierungen bei der Anpassung eines orthopädietechnischen Hilfsmittels auch ohne ein aufwändiges Vermessungssystem zu ermöglichen.

Diese Aufgabe wird mit einem orthopädietechnischen Hilfsmittel der eingangs erwähnten Art dadurch gelöst, dass die Detektoranordnung fest in das orthopädietechnische Hilfsmittel eingebaut oder als auswechselbarer Adapter in das orthopädietechnische Hilfsmittel einbaubar ist und dass die Detektoranordnung einen Abschnitt, der in einer stabilen statischen Belastungsposition in der Messebene drehmomentfrei gelagert ist und der bei geringen Abweichungen von der stabilen Belastungsposition Drehmomente in der Messebene erzeugt, und Indikatoren zur Indikation von in zwei einander entgegen gesetzten Richtungen in der Messebene auftretenden Drehmomente aufweist.

Das erfindungsgemäße orthopädietechnische Hilfsmittel erlaubt eine Überprüfung der Sollausrichtung an dem individuellen Patienten dadurch, dass die Drehmomentfreiheit in der Messebene an den Indikatoren dadurch festgestellt wird, dass in beiden einander entgegen gesetzten Richtungen kein Drehmoment erzeugt wird bzw. dass bei etwaigen Schwankungsbewegungen außerhalb der stabilen statischen Belastungsposition Drehmomente in beiden Richtungen erzeugt werden.

In einer bevorzugten Ausführungsform der Erfindung weist die Detektoranordnung wenigstens einen um einen Drehpunkt schwenkbaren Hebel auf, wobei der Drehpunkt in einer Belastungslinie der stabilen Belastungsposition liegt. Vorzugsweise weist der schwenkbare Hebel den Drehpunkt an einem Ende und ein den drehmomentfrei ausgelegten Abschnitt bildendes drehendes freies Ende auf.

Die Detektoranordnung kann fest in ein orthopädietechnisches Hilfsmittel eingebaut sein. Dabei ist es bevorzugt, wenn das freie Ende im Gebrauch des Hilfsmittels mittels eines Feststellmittels, vorzugsweise einer Feststellschraube, fest mit einem Aufbau des orthopädietechnischen Hilfsmittels verbunden und zur Überprüfung der Sollausrichtung um ein geringes Maß lösbar ist, um eine Verschwenkung des Abschnitts in der Messebene in beiden Richtungen zu ermöglichen. Die Überprüfung der Sollausrichtung des orthopädischen Hilfsmittels geschieht durch Lösen des Feststellmittels, wodurch das freie Ende des Hebels sich bei korrekter Ausrichtung in der vorzugsweise die Sagittalebene bildenden Messebene weder nach oben noch nach unten bewegt, sondern in einer Gleichgewichtsstellung zwischen Anschlägen des gelösten Feststellmittels verharrt. Bei geringfügigen Gleichgewichtsbewegungen des Patienten, also geringfügigen Abweichungen von der stabilen statischen Belastungsposition, wie sie durch Gleichgewichtsbewegungen beim Stehen resultieren, ergibt sich eine entsprechende Bewegung des freien Endes des Hebels in beiden entgegen gesetzten Richtungen. Liegt hingegen eine deutliche Abweichung von der Sollausrichtung, also eine Fehljustierung des orthopädietechnischen Hilfsmittels, vor, wird der Hebel beim Lösen des Feststellmittels fest an einem Anschlag anliegen, wodurch die Fehljustierung erkennbar ist. Nach einer Korrektur der Justierung des orthopädietechnischen Hilfsmittels kann die Überprüfung erneut durchgeführt werden. Wenn dann die Sollausrichtung hergestellt ist, kann das Feststellmittel betätigt und das orthopädietechnische Hilfsmittel in einer korrekten Justierung benutzt werden.

In einer alternativen Ausführungsform kann die Detektoranordnung zwei mittels des Drehpunkts miteinander verbundene Hebel mit freien Enden umfassen, die in der Sollausrichtung während der stabilen Belastungsposition zueinander drehmomentfreie Abschnitte bilden. Die Indikatoren können dabei durch gegen die relative Bewegung der freien Enden wirkende Anschläge gebildet sein.

Um eine merkliche Bewegung der beiden Hebel gegeneinander zu vermeiden, können die Indikatoren elektronische Kraftaufnehmer sein, mit denen die entstehenden Drehmomente messbar sind.

Die Vermeidung elektronischer Bauelemente und deren Energieversorgung gelingt dadurch, dass die Indikatoren elastische Puffer sind, die durch die relative Bewegung der freien Enden verformbar sind. Diese Indikatoren setzen allerdings eine merkbare Bewegung der Hebel zueinander voraus, sodass zweckmäßigerweise die Detektoranordnung als auswechselbare Adapter ausgebildet und in das orthopädische Hilfsmittel nur zur Überprüfung der Sollausrichtung anstelle eines gleich großen Bauelements einbaubar ist. Der Adapter ist dann in das orthopädietechnische Hilfsmittel in einer festen Ausrichtung einsetzbar, um die Sollausrichtung zu überprüfen. Für den Gebrauch des orthopädietechnischen Hilfsmittels wird dann das gleich große Bauelement an der Stelle des Adapters eingebaut, da die Verwendung des orthopädietechnischen Hilfsmittels mit dem Adapter wegen der elastischen Puffer zu einem unsicheren Bewegungserlebnis für den Patienten führen würde.

Bevorzugt ist die Verwendung dieser Ausführungsform für eine Prothese mit einem Kniegelenk. Allerdings ist auch eine Ausbildung als anderes Gelenk, insbesondere Hüftgelenk, möglich.

Die Erfindung soll im Folgenden anhand von in der Zeichnung dargestellten Ausführungsbeispielen näher erläutert werden. Es zeigen:
- Figur 1: einen schematischen Längsschnitt durch einen künstlichen Fuß mit einer durch eine Feststellschraube festgelegten Detektoranordnung;
- Figur 2: die Darstellung gemäß Figur 1 mit gelöster Feststellschraube;
- Figur 3: eine schematische Darstellung einer Prothese mit einer Detektoranordnung oberhalb eines Kniegelenks;
- Figur 4: eine Detaildarstellung der Detektoranordnung gemäß Figur 3.

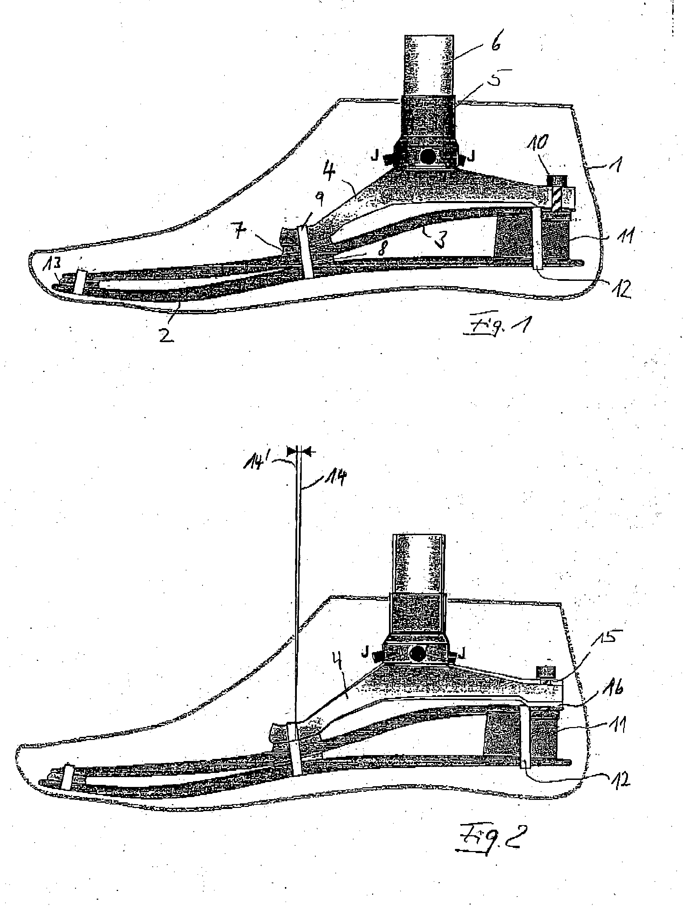

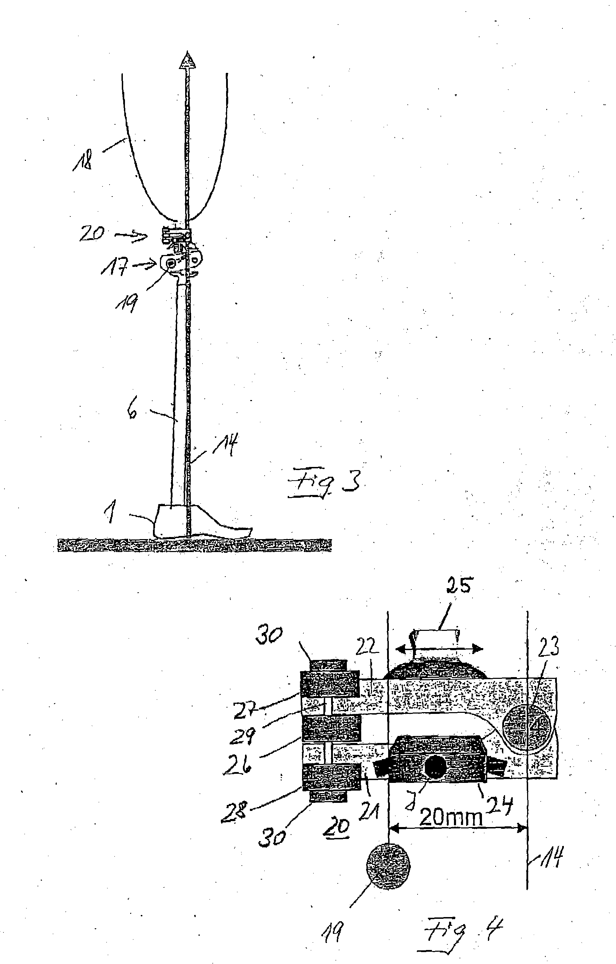

In den Figuren 1 und 2 ist eine Fußprothese dargestellt, deren funktionaler Aufbau in einer die Fußkontur bildenden kosmetischen Hülle 1 untergebracht ist. Im Wesentlichen über die gesamte Länge des Fußes erstreckt sich eine Sohlenstruktur aus einer streifenförmigen unteren Sohlenfeder 2 und einer darüber angeordneten, sich ebenfalls über die Länge des Fußes erstreckende Steuerfeder 3. Beide Federn 2, 3 können aus einem geeigneten elastischen Material bestehen, beispielsweise aus einem kohlefaserverstärkten Kunststoff. Oberhalb der durch die beiden Federn 2, 3 gebildeten Sohlenstruktur befindet sich ein starres Fußoberteil in Form eines Hebels 4, an dessen Oberseite ein (nicht dargestellter) Adapter angeordnet ist, über den eine Justierhülse 5 eines Unterschenkel-Rohrteils 6 ragt. Am unteren Ende der Justierhülse 5 befinden sich vier Justierschrauben J, mit denen die Lage der Justierhülse 5, und damit des Unterschenkel-Rohrteils 6 relativ zu der Fußstruktur einstellbar ist, indem die Justierschrauben J mit Pyramidenstumpfflächen (erkennbar am Adapter gemäß Figur 4) zusammen wirken.

Der Hebel 4 des Fußoberteils erstreckt sich von einem Fersenende zu einem etwa in der Fußmitte liegenden vorderen Ende, an dem der Hebel 4 mit der Steuerfeder 3 und der Sohlenfeder 2 unter Zwischenschaltung jeweils eines flächigen Dämpfers 7, 8 verbunden ist. Die Verbindung mittels eines Bolzens 9 erfolgt so, dass der Hebel 4 relativ zur Steuerfeder 3 und relativ zur Sohlenfeder 2 und die Steuerfeder 3 relativ zur Sohlenfeder 2 eine Schwenkbewegung in der Sagittalebene ausführen kann, während in der senkrecht dazu stehenden Frontalebene eine zumindest verringerte Bewegungsfreiheit gegeben ist.

Am Fersenende ist der Hebel 4 mittels einer Feststellschraube 10 mit dem hinteren Ende der Steuerfeder 3 verbunden. Zwischen dem hinteren Ende der Steuerfeder 3 und dem hinteren Ende der Sohlenfeder 2 ist ein Fersendämpfer 11 in Form eines elastischen Schaumstoffblocks eingesetzt, der beim Fersenauftritt auf Druck und bei Abrollen des Fußes über den Zehenbereich auf Zug belastet wird. Die Zugbelastung ist begrenzt durch einen die Sohlenfeder 2 und die Steuerfeder 3 jeweils außen umschlingenden Gurt 12, der die Kompression des Fersendämpfers 11 nicht behindert, jedoch die Längung des Fersendämpfers unter Zugeinwirkung begrenzt.

Am vorderen Ende im Zehenbereich sind die Sohlenfeder und die Steuerfeder 3 über einen weiteren Dämpfer 13 miteinander verbunden.

Bei einem Fersenauftritt wird der Fersendämpfer 11 komprimiert, wodurch sich die Stellung des Unterschenkelrohrs 6 einer Unterschenkelprothese relativ zu der Sohlenstruktur 2, 3 nach hinten verschwenkt, wodurch eine gewünschte Plantarflektion des Fußes relativ zum Unterschenkelrohr 6 entsteht. Die Steuerfeder 3 wirkt dabei als zweiarmiger Hebel, dessen Fersenabschnitt relativ zum Fersenabschnitt der Sohlenfeder 2 nach unten gedrückt wird, wodurch die Steuerfeder 3 im zum Zehenbereich zeigenden Vorfußbereich die Sohlenfeder 2 anhebt und so eine natürliche Zehenbewegung beim Fersenauftritt imitiert, die das Abrollen des Fußes erleichtert. Beim Abrollen des Fußes über die Standphase hinaus wird die ballig zur Unterseite konvex geformte Sohlenfeder im Vorfußbereich belastet, sodass die Sohlenfeder 2 im Fersenbereich relativ zum Hebel 4 nach unten gedrückt wird, wodurch der Fersendämpfer 11 entlastet bzw. auf Zug belastet wird. Diese Belastung wird durch den flexiblen, aber unelastischen Gurt 12 begrenzt.

Die Konstruktion des dargestellten Fußes beruht darauf, dass sich die durch die Dämpfer 7, 8 und den Bolzen 9 gebildete und einen Drehpunkt ausbildende Gelenkstruktur zwischen Hebel 4 und Sohlenstruktur 2, 3 in der lotrechten Belastungslinie 14 des Patienten beim Stehen befindet, wie dies in Figur 2 dargestellt ist. Demgemäß befindet sich der Schwerpunkt des Patienten lotrecht über dem Drehpunkt 7, 8, 9. Ist dies nicht der Fall, weil sich der Schwerpunkt des Patienten aufgrund der Einstellung der Prothese nicht lotrecht über dem Drehpunkt 7, 8, 9 befindet, sondern mit dieser einen Winkel bildet, wie dies für die Belastungslinie 14' in Figur 2 verdeutlicht ist, wird auf den Hebel 4 ein Drehmoment ausgeübt, sodass der Hebel 4 mit seinem freien Ende gegen den oberen Kopf der Schraube 10 drückt. Befindet sich hingegen die Belastungslinie 14 lotrecht über dem Drehpunkt 7, 8, 9, ist das Fersenende des Hebels 4 unbelastet, also der Hebel 4 bezüglich des Drehpunkts 7, 8, 9 drehmomeritfrei.

Um die korrekte Justierung des Unterschenkel-Rohrteils 6 relativ zum Hebel 4 zu überprüfen, wird die Feststellschraube 10 gelockert, sodass sich für das Fersenende des Hebels 4 ein vertikaler Bewegungsbereich ergibt; der durch einen durch den Schraubenkopf gebildeten oberen Anschlag 15 und einen hier durch das Fersenende der Steuerfeder 3 gebildeten unteren Anschlag 16 begrenzt wird. Befindet sich die lotrechte Belastungslinie 14 lotrecht über dem Drehpunkt 7, 8, 9 ist das Fersenende des Hebels 4 unbelastet und kann daher eine Gleichgewichtsposition innerhalb des durch die Anschläge 15, 16 begrenzten Bewegungsbereichs einnehmen. Mündet die Belastungslinie 14' hingegen unter einem Winkel zur Lotrechten in den Drehpunkt 7, 8, 9 ein, wird auf den Hebel 4 ein Drehmoment ausgeübt, das im Falle der Belastungslinie 14' das Fersenende des Hebels 4 gegen den oberen Anschlag 15 drückt. Das freie Fersenende des Hebels 4 wird daher bei einer entsprechenden Fehljustierung am oberen Anschlag 15 verbleiben, auch wenn der Patient im Stehen die üblichen Gleichgewichts-Ausgleichbewegungen vornimmt.

Ist hingegen die Justierung korrekt, sodass sich der Schwerpunkt innerhalb der lotrechten Belastungslinie 14 befindet, führen die Gleichgewichts-Ausgleichbewegungen des Patienten zu einer Bewegung des Fersenendes des Hebels 4 zwischen dem oberen Anschlag und dem unteren Anschlag, wie dies von einer Hebelwaage bekannt ist. Die bei den Gleichgewichts-Ausgleichsbewegungen, die sich in der Sagittalebene nach vorn und nach hinten ergeben, sind durch eine entsprechende Bewegung des Fersenendes des Hebels 4 in dem Bewegungsbereich nach oben und nach unten erkennbar, wenn die Justierung korrekt erfolgt ist. Andernfalls verbleibt das freie Ende des Hebels 4 an einem der Anschläge 15, 16 wegen der Fehljustierung.

Wenn die richtige Justierung festgestellt worden ist, kann die Feststellschraube 10 angezogen werden, um eine feste Verbindung des Hebels 4 mit der Sohlenstruktur 2, 3 herzustellen und dem Patienten während des Gehens kein Unsicherheitsgefühl durch ein Bewegungsspiel innerhalb des Fußaufbaus zu erzeugen.

Ein anderes Ausführungsbeispiel ist in den Figuren 3 und 4 dargestellt. Erkennbar ist die kosmetische Hülle 1 einer Fußprothese und das daran angeschlossene Unterschenkel-Rohrteil 6. Am oberen Ende des Unterschenkel-Rohrteils 6 ist ein künstliches Kniegelenk 17 angeordnet, das das Unterschenkel-Rohrteil 6 mit einem Aufnahmetrichter 18 für einen Oberschenkelstumpf des Patienten verbindet. In Figur 3 ist die lotrechte Belastungslinie 14 eingezeichnet, die durch den Schwerpunkt des Patienten in der Sagittalebene verlaufen soll. Im Idealfall verläuft die lotrechte Belastungslinie 14 durch den Drehpunkt 7, 8, 9 der Fußprothese und etwa 20 mm vor einer Drehachse 19 des Kniegelenks.

Im Gebrauch ist das Kniegelenk 17 über einen (nicht dargestellten) Befestigungsadapter fest mit dem Aufnahmetrichter 18 verbunden. Der Befestigungsadapter kann dabei mit Zusatzfunktionen ausgerüstet sein. Für die Überprüfung der Justierung der Prothesenanordnung ist zwischen das Kniegelenk 17 und den Aufnahmetrichter 18 ein Adapter 20 mit einer Detektoranordnung eingesetzt. Die Detektoranordnung weist einen unteren Hebel 21 und einen oberen Hebel 22 auf, die an ihren frontalen Enden über ein Drehgelenk 23 miteinander drehbar verbunden sind. Der untere Hebel ist mit einer Adapterbuchse 24 mit Justierschrauben J versehen, um einen justierbaren Anschluss zum Unterschenkel-Rohrteil herzustellen, das an der Oberseite mit einem Adapter mit Kegelstumpfflächen versehen ist. Der obere Hebel 22 verläuft parallel zum unteren Hebel 21 und ist an seiner Oberseite mit einem Adapter 25 versehen, über den der an der Unterseite mit einem Adapter-Buchsenteil (entsprechend 24) zur Verbindung mit dem Adapter 25 versehene Aufnahmetrichter (18) justierbar anschließbar ist.

Das Drehgelenk 23 zwischen den beiden Hebeln 21, 22 ist so angeordnet, dass es sich in der lotrechten Belastungslinie 14, also 20 mm frontal zur Knieachse 19, befindet. Die nach dorsal gerichteten freien Enden der Hebel 21, 22 sind miteinander über einen zwischengeschalteten mittleren Dämpfer 26, einen am oberen Hebel 22 außen angebrachten oberen Dämpfer 27 und einem am unteren Hebel 21 angebrachten äußeren Dämpfer 28 miteinander verbunden, indem eine Zuganordnung 29 mit äußeren Anlagescheiben 30 die Anordnung so zusammenhält, dass die Dämpfer 26 bis 28 berührt, jedoch nicht komprimiert werden. Befindet sich das Drehgelenk 23 mit der Drehachse in der lotrechte Belastungslinie 14, sind die freien Enden der Hebel 21, 22 kräftefrei, sodass die Dämpfer 26 bis 28 unkomprimiert bleiben.

Ist hingegen durch eine Fehljustierung die lotrechte Belastungslinie 14 dorsal von dem Drehgelenk 23 angeordnet, werden die beiden Hebel 21, 22 an ihren freien Enden gegeneinander gedrückt, sodass der mittlere Dämpfer 26 komprimiert wird. Die zu einer Verformung des mittleren Dämpfers 26 führende Kompression ist leicht erkennbar, sodass der mittlere Dämpfer 26 als Indikator für eine Rückwärtsverlagerung der lotrechten Belastungslinie 14 dient.

Befindet sich hingegen die Belastungslinie 14 frontal vor dem Drehgelenk 23, entsteht ein die freien Enden der Hebel 21, 22 voneinander weg drückendes Drehmoment, sodass die äußeren Dämpfer 27, 28 komprimiert - und damit verformt - werden. Die äußeren Dämpfer 27, 28 dienen somit als Indikatoren für eine Vorverlagerung der Belastungslinie 14 vor den Idealzustand.

Es ist ohne weiteres ersichtlich, dass die Dämpfer 26, 28 durch elektronische Kraftaufnehmer (Spannungsaufnehmer, Beschleunigungsaufnehmer) ersetzt werden können, um die auf die Hebel 21, 22 einwirkenden Drehmomente festzustellen. Da diese elektronischen Kraftaufnehmer mit nicht wahrnehmbaren Messwegen arbeiten, kann eine derartige Detektoranordnung in der Prothese während ihrer normalen Benutzung verbleiben, da die Messanordnung nicht zu einem Spiel in der Prothesenanordnung führt.

Die einfachere und preiswertere Ausbildung der Detektoranordnung mit den Dämpfern 26 bis 28 erfordert hingegen den Austausch der Detektoranordnung gegen einen Adaptereinsatz für die normale Nutzung der Prothesenanordnung.

Das Drehgelenk 23 kann in einer einfachen Ausführungsform auch durch ein Festkörperscharnier gebildet sein. Die Indikatoren können hierbei auch durch Schalter gebildet werden.

## Patentansprüche

1. Orthopädietechnisches Hilfsmittel zur Überprüfung der mit einem justierbaren Element herstellbaren Sollausrichtung an einem individuellen Patienten mit Hilfe einer Detektoranordnung, **dadurch gekennzeichnet, dass** die Detektoranordnung fest in das orthopädietechnische Hilfsmittel eingebaut oder als auswechselbarer Adapter (20) in das orthopädietechnische Hilfsmittel einbaubar ist und dass die Detektoranordnung einen Abschnitt, der in einer stabilen statischen Belastungsposition in der Messebene drehmomentfrei gelagert ist und der bei geringen Abweichungen von der stabilen Belastungsposition Drehmomente in der Messebene erzeugt, und Indikatoren (15, 16; 26, 27, 28) zur Indikation von in zwei einander entgegen gesetzten Richtungen in der Messebene auftretenden Drehmomente aufweist.

2. Orthopädietechnisches Hilfsmittel nach Anspruch 1, **dadurch gekennzeichnet, dass** die Detektoranordnung wenigstens einen um einen Drehpunkt (7, 8, 9; 23) schwenkbaren Hebel (4; 21, 22) aufweist und dass der Drehpunkt (7, 8, 9; 23) in einer Belastungslinie (14) der stabilen Belastungsposition liegt.

3. Orthopädietechnisches Hilfsmittel nach Anspruch 2, **dadurch gekennzeichnet, dass** der Hebel (4; 21, 22) den Drehpunkt (7, 8, 9; 23) an einem Ende und ein den Abschnitt bildendes drehendes freies Ende aufweist.

4. Orthopädietechnisches Hilfsmittel nach Anspruch 3, **dadurch gekennzeichnet, dass** das freie Ende im Gebrauch des Hilfsmittels mittels eines Feststellmittels (10) fest mit einem Aufbau des orthopädietechnischen Hilfsmittels verbunden und zur Überprüfung der Sollausrichtung um ein geringes Maß lösbar ist, um eine Verschwenkung des Abschnitts des Hebels (4) in der Messebene in beiden Richtungen zu ermöglichen.

5. Orthopädietechnisches Hilfsmittel nach Anspruch 4 in Form einer Fußprothese.

6. Orthopädietechnisches Hilfsmittel nach einem der Ansprüche 1 bis 4, **dadurch gekennzeichnet, dass** die Detektoranordnung zwei mittels des Drehpunktes (23) miteinander verbundene Hebel (21, 22) mit freien Enden umfasst, die in der Sollausrichtung während der stabilen Belastungsposition zueinander drehmömentfreie Abschnitte bilden und dass die Indikatoren durch gegen die relative Bewegung der freien Enden wirkende Anschläge (26, 27, 28) gebildet sind.

7. Orthopädietechnisches Hilfsmittel nach Anspruch 6, **dadurch gekennzeichnet, dass** die Indikatoren elektronische Kraftaufnehmer sind.

8. Orthopädietechnisches Hilfsmittel nach Anspruch 6, **dadurch gekennzeichnet, dass** die Indikatoren elastische Dämpfer (26, 27, 28) sind, die durch die relative Bewegung der freien Enden verformbar sind.

9. Orthopädietechnisches Hilfsmittel nach einem der Ansprüche 1 bis 3 und 5 bis 8, **dadurch gekennzeichnet, dass** die Detektoranordnung als auswechselbare Adapter (20) ausgebildet und in das orthopädietechnische Hilfsmittel nur zur Überprüfung der Sollausrichtung anstelle eines gleich großen Bauelements einbaubar ist.

10. Orthopädietechnisches Hilfsmittel nach einem der Ansprüche 6 bis 9, **dadurch gekennzeichnet, dass** es eine Prothese mit einem Kniegelenk (17) ist.

## Claims

1. An orthopedic aid for checking by means of a detector arrangement at an individual patient a desired orientation obtainable by means of an adjustable element **characterized in that** the detector arrangement is fixedly mounted in the orthopedic aid or can be mounted in the orthopedic aid as an exchangeable adapter (20), and that the detector arrangement includes a section being torque-free in the measuring plane during a stable static load position and generating torques in the measuring plane during slight deviations from the stable load position, and including indicators (15, 16; 26, 27, 28) for indicating torques arising in two mutually opposite directions in the measuring plane.

2. The orthopedic aid as claimed in claim 1,**characterized in that** the detector arrangement has at least one lever (4; 21, 22) that can pivot about a pivot point (7, 8, 9; 23), and **in that** the pivot point (7, 8, 9; 23) lies in a load line (14) of the stable load position.

3. The orthopedic aid as claimed in claim 2, **characterized in that** the lever (4; 21, 22) has the pivot point (7, 8, 9; 23) at one end, and a rotating free end forming the section.

4. The orthopedic aid as claimed in claim 3, **characterized in that**, during use of the aid, the free end is securely connected with the aid of a locking means (10) to a structure of the orthopedic aid and, for verification of the desired orientation, can be released slightly in order to permit a pivoting of the section of the lever (4) in the measuring plane in both directions.

5. The orthopedic aid as claimed in claim 4, in the form of a foot prosthesis.

6. The orthopedic aid as claimed in one of claims 1 through 4, **characterized in that** the detector arrangement comprises two levers (21, 22) with free ends which are connected to each other by means of the pivot point (23) and which, in the desired orientation during the stable load position, form torque-free sections, and **in that** the indicators are formed by limit stops (26, 27, 28) that act counter to the relative movement of the free ends.

7. The orthopedic aid as claimed in claim 6, **characterized in that** the indicators are electronic force transducers.

8. The orthopedic aid as claimed in claim 6, **characterized in that** the indicators are elastic dampers (26, 27, 28) that can be deformed by the relative movement of the free ends.

9. The orthopedic aid as claimed in one of claims 1 through 3 and 5 through 8, **characterized in that** the detector arrangement is designed as an exchangeable adapter (20) and can be fitted into the orthopedic aid only for the verification of the desired orientation, in place of a structural element of the same size.

10. The orthopedic aid as claimed in one of claims 6 through 9, **characterized in that** it is a prosthesis with a knee joint (17).

## Revendications

1. Accessoire technique orthopédique pour contrôler l'orientation de consigne qui peut être établie avec un élément ajustable sur un patient individuel avec l'aide d'un agencement détecteur, **caractérisé en ce que** l'agencement détecteur est fermement intégré dans l'accessoire technique orthopédique ou bien peut être intégré dans l'accessoire technique orthopédique sous la forme d'un adaptateur interchangeable (20), et **en ce que** l'agencement détecteur comprend une portion qui, dans une position de chargement statique stable, est située dans le plan de mesure en étant exempt de torque et qui, en cas de faibles écarts depuis la position de chargement stable, engendre des torques dans le plan de mesure, et des indicateurs (15, 16; 26, 27, 28) pour l'indication des torques qui apparaissent dans le plan de mesure dans deux directions mutuellement opposées.

2. Accessoire technique orthopédique selon la revendication 1, **caractérisé en ce que** l'agencement détecteur comprend au moins un levier (4 ; 21, 22) capable de pivoter autour d'un centre de rotation (7, 8, 9 ; 23), et **en ce que** le centre de rotation (7, 8, 9 ; 23) est situé dans une ligne de chargement (14) de la position de chargement stable.

3. Accessoire technique orthopédique selon la revendication 2, **caractérisé en ce que** le levier (4 ; 21, 22) comprend le centre de rotation (7, 8, 9 ; 23) à une extrémité, et une extrémité libre rotative qui forme la portion.

4. Accessoire technique orthopédique selon la revendication 3, **caractérisé en ce que** l'extrémité libre est reliée, lors de l'utilisation de l'accessoire, au moyen d'un élément d'immobilisation (10), fermement avec une superstructure de l'accessoire technique orthopédique et est susceptible d'être légèrement détachée pour contrôler l'orientation de consigne, afin de permettre un pivotement de la portion du levier (4) dans le plan de mesure dans les deux directions.

5. Accessoire technique orthopédique selon la revendication 4, sous la forme d'une prothèse de pied.

6. Accessoire technique orthopédique selon l'une des revendications 1 à 4, **caractérisé en ce que** l'agencement détecteur comprend deux leviers (21, 22) reliés l'un à l'autre au moyen du centre de rotation (23) avec des extrémités libres qui forment, dans l'orientation de consigne pendant la position de chargement stable, des portions exemptes de torque l'une par rapport à l'autre, et **en ce que** les indicateurs sont formés par des butées (26, 27, 28) agissant à l'encontre du mouvement relatif des extrémités libres.

7. Accessoire technique orthopédique selon la revendication 6, **caractérisé en ce que** les indicateurs sont des capteurs de force électroniques.

8. Accessoire technique orthopédique selon la revendication 6, **caractérisé en ce que** les indicateurs sont des amortisseurs élastiques (26, 27, 28) qui sont déformables en raison du mouvement relatif des extrémités libres.

9. Accessoire technique orthopédique selon l'une des revendications 1 à 3 et 5 à 8, **caractérisé en ce que** l'agencement détecteur est réalisé sous forme d'adaptateur interchangeable (20), et n'est susceptible d'être intégré dans l'accessoire technique orthopédique que pour le contrôle de l'orientation de consigne au lieu d'un élément structurel de même taille.

10. Accessoire technique orthopédique selon l'une des revendications 6 à 9, **caractérisé en ce qu'**il est une prothèse avec une articulation du genou (17).
